# EUROPEAN PATENT APPLICATION

(11) **EP 2 517 702 A1**
(43) Date of publication of application: **31.10.2012**
(21) Application number: 12167951.8
(22) Date of filing: 12.10.2006
(51) Int. Cl.: A61K 31/19, A61K 31/353, A61K 8/37

(54) **Compounds, compositions and methods to treat skin diseases characterized by cellular proliferation and angiogenesis**

(30) Priority: 27.04.2006 US 412618
(62) Divisional of application: 10191108.9
(71) Applicant: Paloma Pharmaceuticals, Inc., Jamaica Plain, MA 02130 (US)
(72) Inventor: Sherris, David, Jamaica Plain, MA Massachusetts 02130 (US)
(74) Representative: Duffy, Assumpta Dympna

(57) **Abstract**

Described herein are compounds, compositions and methods for preventing and/or treating skin diseases including, but not limited to, psoriasis and atopic dermatitis as well as providing anti-aging benefits which results in reduced appearance of wrinkles and aged skin, improved skin color, treatment of photodamaged skin, improvement in skin's radiance and clarity and finish, and an overall healthy and youthful appearance of the skin, involving aberrant angiogenesis and hyperplasia employing one for more benzo[c]chromen-6-one derivatives.

## Description

### RELATED APPLICATIONS

This patent application claims priority to and the benefit of US Patent Application Serial No. 11/412,618, filed April 27, 2006 which claims priority to and the benefit of US Provisional Application Serial No. 60/675,707, filed April 28, 2005.

### FIELD OF THE INVENTION

The present invention relates to compositions and methods for preventing and/or treating skin diseases associated with cellular proliferation and/or angiogenesis. The current invention is directed in part to a series of benzo(c)chromen-6-one chemical compositions that demonstrate therapeutic benefit in diseases involving abnormal cellular proliferation, abnormal angiogenesis or a combination thereof.

### BACKGROUND OF THE INVENTION

Blood vessels that make up the cardiovascular system may be broadly divided into arteries, veins and capillaries. Arteries carry blood away from the heart at relatively high pressure; veins carry blood back to the heart at low pressure, while capillaries provide the link between the arterial and venous blood supply. During embryonic development, vessels are first formed through vasculogenesis, utilizing pluripotent endothelial cell precursors. Later, through arteriogenesis, larger blood vessels are formed possessing a more complex structure of endothelial cells, smooth muscle cells and pericytes (tunica media). Although arteriogenesis is not considered to occur in the adult, blood vessels may be formed in the adult through vasculogenesis and notably a process known as angiogenesis. Under normal conditions, angiogenic neovascularization occurs during such conditions as wound repair, ischemic restoration and the female reproductive cycle (generating endometrium forming the corpus luteum and during pregnancy to create the placental). The capillaries, relatively simple vessels formed by angiogenesis, lack a developed tunica as they are predominantly composed of endothelial cells and to a lesser extent perivascular cells and basement membrane.

Psoriasis and atopic dermatitis show similarities in that they both display a chronic inflammatory state characterized by keratinocyte hyperplasia, angiogenesis, and infiltration with mononuclear cells, which could include activated T cells, mast cells and monocytes. Skin is vascularized at the dermal subcutaneous border and directly beneath the epidermal layer producing an interconnected network. In psoriasis and atopic dermatitis, this vascular network expands with a newly forming aberrant neovasculature. Angiogenic stimuli, notably VEGF and other angiogenic cytokines, initially originate from the epidermis and are further supported via immune cells invading through expanded vascularization. High levels of VEGF have been found in the epidermis of psoriatic lesions which is believed to play an important role in the pathogenesis of psoriasis. Early on, psoriatic and atopic dermatitis skin will utilize nearby normal vessels to provide nutrients and oxygen. However, as these skin diseases develop, the skin induces angiogenesis to create additional vascular support. Capillaries in psoriatic plaques are elongated, tortuous and dilated and show increased endothelial cell proliferation not unlike that seen in atopic dermatitis. Normally, angiogenesis is kept in check by the body naturally creating angiogenic inhibitors to counteract angiogenic factors. However, the altered skin tissue changes this balance by producing angiogenic growth factors through inherent sources, the fibroblasts or keratinocytes, or infiltration of cells which could provide such factors in excess of the angiogenic inhibitors, thus favoring blood vessel growth. Skin disease initiated angiogenesis is not unlike angiogenesis observed during normal vessel growth. Angiogenic factors pass from the abnormal skin or cellular infiltrates to the normal endothelium, binding the endothelial cell, activating it and inducing endothelial signaling events leading to endothelial cell proliferation. Endothelial tubes begin to form, homing in toward the abnormal skin with the formation of capillary loops. Capillaries then undergo a maturation process to stabilize loop structure.

The skin diseases, psoriasis and atopic dermatitis, are but one condition associated with a pathological neovasculature. Photodamaged skin is characterized clinically by coarseness, telangiectasia (dilation of capillaries causing elevated dark red blotches), wrinkling, discrete hyperpigmented and hypopigmented areas, atrophy, and ultimately the development of neoplasms. Photodamaged skin also displays characteristics of increased angiogenesis and cell infiltration.

Angiogenesis may be considered a key component in the pathogenesis of a skin disease and photodamaged skin. If through therapeutic intervention angiogenesis could be slowed down or eliminated, anti-angiogenic agents would then be expected to abolish or lessen a variety of neovasculature associated skin diseases and photodamaged skin. Anti-angiogenic therapy will likely be very effective at suppressing aberrant skin tissue by denying the skin a blood supply thereby inhibiting the infiltration of cells causing both inflammation and increased vascularization. Furthermore, dual anti-angiogenic activity along with anti-proliferative activity to inhibit epidermal keratinocyte hyperplasia would also be expected to help alleviate the damage caused by such skin diseases and photodamage.

Thus, there remains a need to develop agents that demonstrate anti-proliferative effects against human endothelial cells for the treatment of a variety of skin diseases and photodamaged aging skin. In addition to endothelial cells within diseased or aging skin, an inhibitory effect directly on proliferating keratinocyte cells for the treatment of skin diseases or aging skin, or other cells acting as an initiator of angiogenesis for skin diseases and aging skin could further be beneficial. The present invention seeks to meet these and other needs.

### BRIEF SUMMARY OF THE INVENTION

The present invention relates to compositions and methods for treating skin diseases associated with cellular proliferation and/or angiogenesis. Skin diseases that are the object of the present invention include, but not limited to, psoriasis and atopic dermatitis, and aging skin to improve or prevent the condition of wrinkled, lined, dry, flaky, aged or photodamaged skin and improving skin thickness, elasticity, flexibility, radiance glow and plumpness, which method includes applying said compounds to the skin either applied prophylactically to normal healthy skin to prevent or reduce the deteriorative changes or to pre-existing aged skin, associated with cellular proliferation and/or angiogenesis. The current invention is directed in part to a series of chemical compositions that demonstrate a therapeutic benefit in diseases involving abnormal cellular proliferation, abnormal angiogenesis or a combination thereof.

One embodiment of the present invention is directed to compositions used to prevent and/or treat abnormal cellular proliferation associated with skin disease. In one aspect, the invention is directed to a series of benzo[c]chromen-6-one derivatives that demonstrate enhanced anti-proliferative effects against human endothelial cells for the treatment of a variety of skin diseases and aging skin.

In another embodiment, the present invention is directed toward methods of administering a therapeutically effective amount of one or more benzo[c]chromen-6-one derivative compositions described herein to a subject in need thereof. In one aspect, the targeted subject has been diagnosed with or is predisposed toward one or more skin disease associated with abnormal cellular proliferation/angiogenesis (including aging skin).

Other features and advantages of the invention will be apparent from the following detailed description of embodiments thereof.

### BRIEF DESCRIPTION OF THE DRAWINGS

Figure 1 is a bar graph showing data from an assay conducted to illustrate the efficacy of inhibition of endothelial cell proliferation by compounds of the present invention;
Figure 2 is a bar graph presenting data on the apoptotic inducing ability of the compounds of the present invention; and
Figure 3 is a bar graph presenting data on the apoptotic inducing activity in keratinocytes.

### DETAILED DESCRIPTION OF THE INVENTION

The present invention relates to compositions and methods for preventing and/or treating skin diseases associated with unwanted cellular proliferation and/or angiogenesis. The current invention is directed in part to a series of chemical compositions that demonstrate therapeutic benefit in diseases involving abnormal cellular proliferation, abnormal angiogenesis or a combination thereof. In a particular aspect, the instant invention relates to benzo[c]chromen-6-one derivatives that demonstrate their effect on diseases characterized by abnormal proliferation, abnormal angiogenesis or a combination thereof.

The term "derivative" is understood by those skilled in the art. For example, a derivative can be understood as a chemical compound that is produced from another compound of similar structure in one or more steps, such as illustrated in Table I (*infra*) for benzo[c]chromen-6-one.

Skin disease therapeutic agents currently under development are based on a variety of targeting strategies. One strategy is the use of natural inhibitors of angiogenesis such as thrombospondin. Another strategy is the use of agents that block receptors required to stimulate angiogenesis, such as antagonists of the VEGF receptor.

Angiogenesis is an attractive therapeutic target for skin diseases and aging skin due to its selectivity of action. Blood vessels in growing skin diseases and aging skin are in a microenvironment conducive to cellular activation and rapid proliferation whereas blood vessels in most normal tissues are quiescent. This microenvironment inducing cellular activation and rapid proliferation are believed to be the physiological differences that allow the selective targeting of blood vessels in the aberrant skin by anti-angiogenic agents.

The present invention relates to a therapeutic formulation comprising one or more compositions useful in the treatment of unwanted cellular proliferation and/or angiogenesis and/or keratinocytes proliferation.

In accordance with the present invention, there is provided a pharmaceutical composition comprising a therapeutically effective amount of one or more benzo[c]chromen-6-one derivatives having the following of structure depicted in Table I:

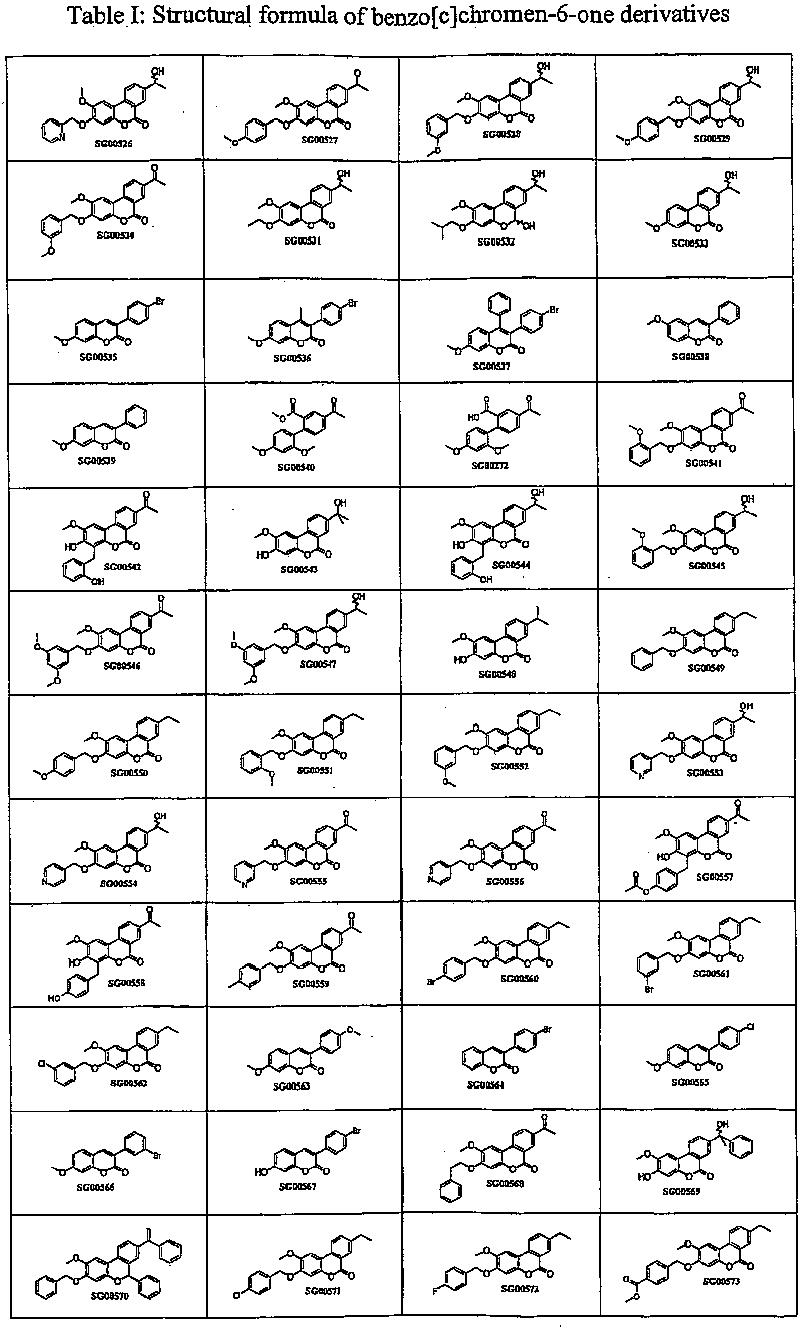

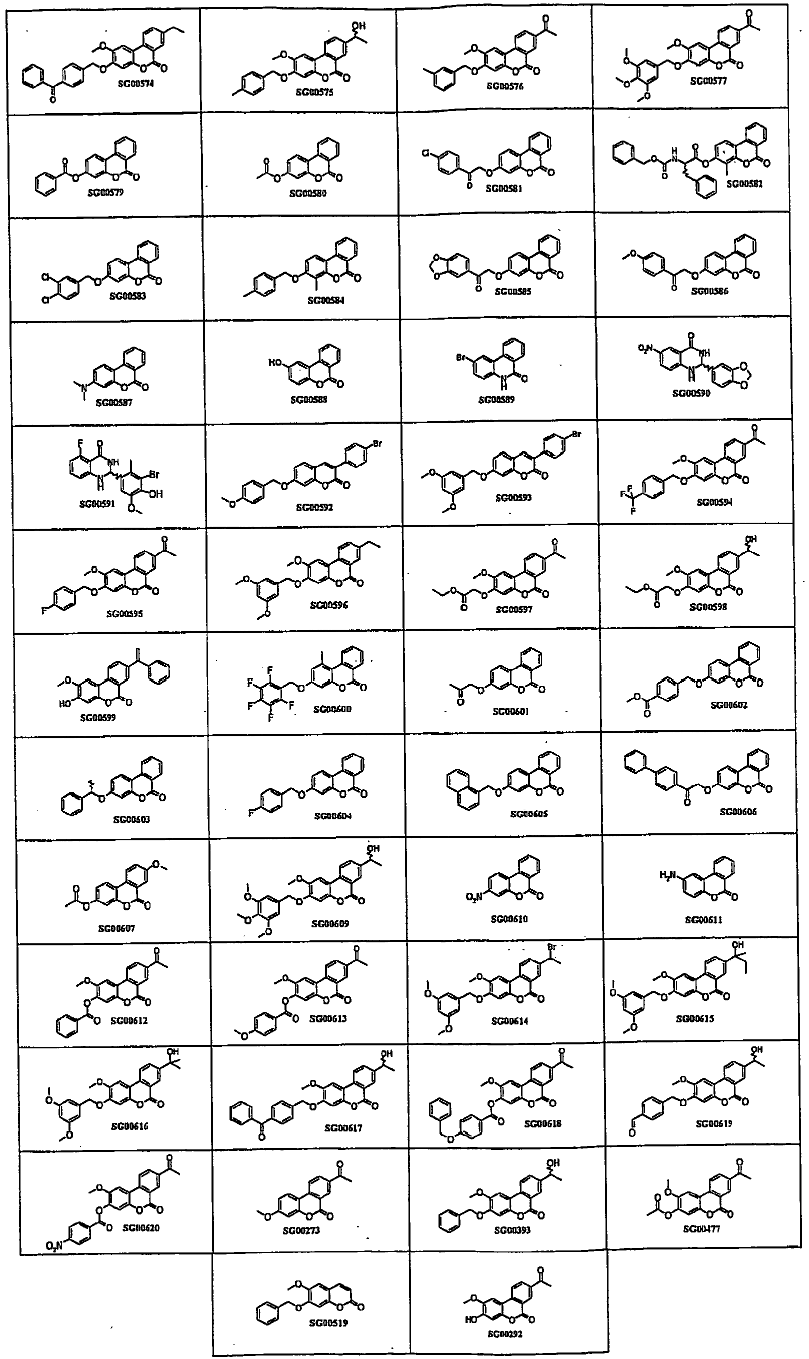

The compounds of Table I exhibit anti-angiogenic and/or anti-keratinocyte activities. Those skilled in the art will appreciate that the invention includes other benzo[c]-chromen-6-one derivatives having anti-cellular proliferative, and/or anti-angiogenic, and/or anti-keratinocyte activities. These characteristics can be determined for each test derivative using the assays detailed below and elsewhere in the literature.

The process or processes by which benzo[c]chromen-6-one derivates affect cell growth remains to be fully researched, however, benzo[c]chromen-6-one derivates may induce changes in the levels and activities of various proteins involved in the progression of the cell cycle. These include cofactors of DNA replication and repair, *e*.*g*., proliferating cell nuclear antigen and cell division cycle kinases (and regulators). Benzo[c]chromen-6-one may also up-regulate Death Receptor 5 and caspase 8.

Assays relevant to these mechanisms of action and inhibition of cell proliferation are well-known in the art. For example, anti-mitotic activity mediated by effects on tubulin polymerization activity can be evaluated by testing the ability of a benzo[c]chromen-6-one derivative to inhibit tubulin polymerization and microtubule assembly *in vitro.* Other such assays include counting of cells in tissue culture plates or assessment of cell number through metabolic assays or incorporation into DNA of labeled (radio-chemically, *e*.*g*., ³H-thymidine or fluorescently labeled) or immuno-reactive (BrdU) nucleotides. In addition, anti-angiogenic activity may be evaluated through endothelial cell migration, endothelial cell tubule formation or vessel outgrowth in *ex-vivo* models of rat aortic rings.

The present invention also relates to topical application, implants or other devices comprised of one or more compositions described herein or prodrugs thereof wherein the composition or prodrug is formulated in a biodegradable or non-biodegradable format for sustained release. Non-biodegradable formats release the drug in a controlled manner through physical or mechanical processes without the format being itself degraded. Bio-degradable formats are designed to gradually be hydrolyzed or solubilized by natural processes in the body, allowing gradual release of the admixed drug or prodrug. Both bio-degradable and non-biodegradable formats and the process by which drugs are incorporated into the formats for controlled release are well known to those skilled in the art. These topical applications, implants or devices can be implanted in the vicinity where delivery is desired, for example, at the site of a aberrant skin or in the vicinity of aberrant vasculature.

The compositions of the present invention can be associated with an implant or device. The association can be facilitated through the conjugation of the composition to the implant or device (on the interior and/or exterior surface), the composition can be sequestered within the implant or device and the like. This conjugation can be covalent or noncovalent. The conjugation can be hydrolysable. One skilled in the art is well aware of the various ways to facilitate this conjugation.

The present invention also relates to conjugated prodrugs and uses thereof. More particularly, the invention relates to conjugates of benzo[c]chromen-6-one derivatives and the use of such conjugates in the prophylaxis or treatment of conditions associated with uncharacteristic cell proliferation and/or uncharacteristic angiogenesis. Such diseases include, but are not limited to, excessive, abnormal stimulation or proliferation of keratinocytes, endothelial cells or other pathologically involved cells.

The present invention also provides a conjugated prodrug of a benzo[c]chromen-6-one derivative conjugated to a biological activity modifying agent, *e.g*., a peptide, an antibody or fragment thereof, or *in vivo* hydrolysable esters, such as methyl esters, phosphate or sulfate groups, and amides or carbamates. The incorporation of benzo[c]chromen-6-one derivatives into a disease-dependently activated prodrug enables significant improvement of potency and selectivity in treating one or more disease conditions referred to hereinabove.

In addition to the compounds of the present invention, the pharmaceutical composition of this invention may also contain or be co-administered (simultaneously or sequentially) with one or more pharmacological agents of value in treating one or more disease conditions referred to hereinabove. Such agents include, but are not limited to, pharmaceutical agents well known to those skilled in the art for their anti-endothelial cell or anti-keratinocyte activity, additionally, anti-inflammatory agents well known to those skilled in the art.

Furthermore, the benzo[c]chromen-6-one derivatives or prodrugs thereof may be incorporated into bio-degradable or non-degradable formats allowing for sustained release. For example, the formulation being implanted in the proximity of where the delivery is desired, at the site of the skin disease or aging skin or in the vicinity of aberrant vasculature. Alternatively, the pharmaceutical formulation can be packaged into a delivery vehicle that has a chemical moiety that provides for specificity. For example, the moiety can be an antibody or some other such molecule that directs and facilitates delivery of the active agent to the desirable site (skin disease or aging skin) - such as a ligand known to those skilled in the art that interacts with one or more receptors of interest.

The present invention also relates to the provision of a pharmaceutical composition comprising benzo[c]chromen-6-one derivatives or prodrugs thereof according to the present invention together with a pharmaceutical acceptable carrier, diluent or excipient.

The present invention also pertains to methods of prophylaxis or treatment of a condition associated with any skin disease or aging skin characterized by uncharacteristic cell proliferation and/or uncharacteristic angiogenesis and/or inflammation, said method including administering to a subject in need of such prophylaxis or treatment an effective amount of benzo[c]chromen-6-one derivatives or prodrugs thereof according to the present invention as described hereinabove. (It should be understood that prophylaxis or treatment of said condition includes amelioration of said condition.)

By "effective amount" it is meant a therapeutically effective amount that relieves symptoms, partially or completely, associated with a particular disease or syndrome. Such amounts can be readily determined by an appropriately skilled practitioner, taking into account the condition to be treated, the route of administration, and other relevant factors - well known to those skilled in the art. Such a person will be readily able to determine a suitable dose, mode and frequency of administration.

Pharmaceutically acceptable salts of the benzo[c]chromen-6-one derivatives or prodrugs thereof may be prepared in any conventional manner. *In vivo* hydrolysable esters, for example, methyl esters, phosphate or sulfate groups, and amides or carbamates may be prepared in any conventional manner.

The benzo[c]chromen-6-one derivatives or prodrugs thereof can be provided as physiologically acceptable formulations using known techniques and these formulations can be administered by standard routes. The compositions may be administered through means including, but not limited to, topical, oral, rectal or parenteral, for example, intravenous, subcutaneous or intramuscular, route. In addition, the compositions may be incorporated into formats allowing for sustained release, the formats being implanted in the proximity of where the delivery is desired, for example, at the site of the skin disease or aging skin or in the vicinity of aberrant vasculature. The dosage of the composition will depend on the condition being treated, the particular derivative used, and other clinical factors such as weight and condition of the subject and the route of administration of the compound - all of which is appreciated by those skilled in the art. For example, a person skilled in the art will be able by reference to standard texts, such as Remington's Pharmaceuticals Sciences 17^{th} edition (the entire teaching of which is incorporated herein by reference), determine how the formulations are to be made and how these may be administered.

The formulations including, but not limited to, those suitable for oral, rectal, nasal, inhalation, topical (including, but not limited to, dermal, transdermal, buccal and sublingual), vaginal or parenteral (including, but not limited to, subcutaneous, intramuscular, intravenous, intradermal, and inhalation administration. The formulations may be conveniently presented in unit dosage form and may be prepared by conventional pharmaceutical techniques. Such techniques include the step of bringing into association the active ingredient and a pharmaceutical carrier(s) or excipient(s). The formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion, etc.

A tablet may be made by compression or molding, optimally with one or more accessory ingredient. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tablets may be made by molding, in a suitable machine, a mixture of the powdered compound moistened with an inert liquid diluent. The tablets may optionally be coated or scored and may be formulated so as to provide a slow or controlled release of the active ingredient therein.

Formulations suitable for administration via the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutical, cosmeceutical or cosmetic acceptable carrier. A viable delivery system is a transdermal patch containing the ingredient to be administered.

The composition according to the invention also comprises a pharmaceutical or cosmetically acceptable vehicle to act as a diluent, dispersant or carrier for the benzo[c]chromen-6-one derivatives or prodrugs in the composition, so as to facilitate its distribution when the composition is applied to the skin.

Vehicles other than or in addition to water can include liquid or solid emollients, solvents, humectants, thickeners and powders. In one aspect, the nonaqueous carrier is a polydimethyl siloxane and/or a polydimethyl phenyl siloxane. Silicones of this invention may be those with viscosities ranging anywhere from about 10 - 10,000 mm² /s (centistokes) at 25° C. Especially desirable are mixtures of low and high viscosity silicones. These silicones are available from the General Electric Company under the 200 to 550 series. Amounts of silicone which can be utilized in the compositions of this invention range anywhere from 5% to 95%, and from 25% to 90% by weight of the composition.

The pharmaceutical or cosmetically acceptable vehicle will usually form from 5% to 99.9%, in one aspect, from 25% to 80% by weight of the composition, and can, in the absence of other cosmetic adjuncts, form the balance of the composition. In one aspect, the vehicle is a least 80 wt. % water, by weight of the vehicle. In another aspect, water comprises at least 50 wt. % of the inventive composition, and from 60 to 80 wt. %, by weight of the composition.

In one embodiment of the invention, compositions also include an alpha-hydroxy acid. Hydroxyacids increase desquamation of normal skin resulting in smoother, younger looking skin. The hydroxyacid can be chosen from alpha-hydroxy acids, beta-hydroxyacids (e.g. salicylic acid), other hydroxycarboxylic acids (e.g. dihydroxycarboxylic acid, hydroxyl-dicarboxylic, hydroxtricarboxylic) and mixtures thereof or combination of their stereoisomers (DL, D or L).

The compositions containing benzo[c]chromen-6-one derivatives or prodrugs can include glycolic acid and/or lactic acid because they have been shown to be particularly efficacious at delivering cosmetic benefits from those knowledgeable in the art.

In another aspect, the hydroxyl acid is chosen from lactic acid, 2-hydroxyoctanoic acid, hydroxylauric acid glycolic acid, and mixtures thereof.

It is to be understood that depending on the pH of the composition, the hydroxyl acid may be present as a salt, e.g. ammonium, potassium, or sodium salt. The compositions may have any pH in the general range of 2.5 to 10.

An oil or oily material may be present, together with an emulsifier to provide either a water-in-oil emulsion or an oil-in-water emulsion, depending largely on the average hydrophilic-lipophilic balance of the emulsifier employed.

The compositions can include sunscreens. Sunscreens include those materials commonly employed to block ultraviolet light. Illustrative compounds are the derivatives of PABA, cinnamate and salicylate. For examply, octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks Parsol MCX and Benzophenone-3, respectively. The exact amount of sunscreen employed in the emulsions can vary depending upon the degree of protection desired from the sun's UV radiation.

Emollients are often incorporated into pharmaceutical or cosmetic compositions. Levels of such emollients may range from 0.5% to 50%, or can be between 5% and 30% by weight of the total composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and diocytl succinate. Acceptable branched chain fatty esters include 2-ethyl-hexyl myristate, isopropyl stearate and isosteryl palmitate. Acceptable tribasic acid esters include triisopropyl trilinoleate and trilauryl citrate. Acceptable straight chain fatty esters include lauryl palmitate, myristyl lactate, and stearyl oleate. Suitable esters include coco-caprylate/caprate (a blend of coco-caprylate and coco-caprate), propylene glycol myristyl ether acetate, diisopropyl adimate and cetyl octanoate.

Suitable fatty alcohols and acids include those compounds having from 10 to 20 carbon atoms - such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as poly-propylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within cosmetic compositions are thickeners. A thickener will usually be present in amounts anywhere from 0.1 to 20% by weight, from about 0.5% to 10% by weight of the composition. Exemplary thickeners are cross-linked polyacrylate materials available under the trademark Carbopol form B.F. Goodrich Company. Gums may also be employed such as xanthan, carrageenan, gelatin, karaya, pectin and locust beans gum. Under certain circumstances the thickening function may be accomplished by a material also serving as a silicone or emollient. For instance, silicone gums in excess of 10 centistokes and esters such as glycol stearate have dual functionality.

Powders may be incorporated into pharmaceutical or cosmetic compositions. These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into pharmaceutical or cosmetic compositions. These ingredients may include coloring agents, opacifiers and perfumes. Amounts of these other adjunct minor components may range anywhere from 0.001 % up to 20% by weight of the composition.

The pharmaceutical or cosmetic composition is primarily intended as a product of topical application to skin, especially as an agent for conditioning, moisturizing and smoothing the skin, and preventing or reducing the appearance of lined, wrinkled or aged skin. In use, a small quantity of the composition, for example from 1 to 100 mL is applied to exposed areas of the skin, from a suitable container or applicator and, if necessary, it is then spread over and/or rubbed into the skin using the hand or fingers or a suitable device. The topical skin treatment compositions can be formulated as a lotion, a cream or a gel. The composition can be packaged in a suitable container to suit its viscosity and intended use by the consumer. For example, a lotion or cream can be packaged in a bottle or a roll-ball applicator, or a propellant-driven aerosol device or a container fitted with a pump suitable for finger operation. When the composition is a cream, it can be simply be stored in a non-deformable bottle or squeeze container, such as a tube or a lidded jar. The composition may also be included in capsules.

Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is taken, for example, by rapid inhalation through the nasal passage from a container of the powder held close up to the nose. Suitable formulations include wherein the carrier is a liquid for administration, as for example a nasal spray or as nasal drop, including aqueous or oily solutions of the active ingredient.

Formulations suitable for vaginal administration may be presented as pessaries, tampons, creams, gels, pastes, foams or spray formulations containing, in addition to the active ingredient, ingredients such as carriers as are known in the art to be appropriate.

Formulation suitable for inhalation may be presented as mists, dusts, powders or spray formulations containing, in addition to the active ingredient, ingredients such as carriers as are known in the art to be appropriate.

Formulations suitable for parenteral administration include aqueous and non-aqueous sterile injection solutions which may contain anti-oxidants, buffers, bacteriostatic agents and solutes which render the formulation isotonic with the blood of the intended recipient; and aqueous and non-aqueous sterile suspensions which may include suspending agents and thickening agents. The formulations may be presented in unit-dose or multidose containers, for example, sealed ampules and vials, and may be stored in a freeze-dried, lyophilized, conditions requiring only the addition of the sterile liquid, for example, water for injections, immediately prior to use. Extemporaneous injection solution and suspensions may be prepared from sterile powders, granules and tablets of the kinds previously described.

Acceptable unit dosage formulations are those containing a daily dose or unit, daily sub-dose, as herein above recited, or an appropriate fraction thereof, of the administered ingredient.

In addition to the ingredients mentioned above, the formulations of the present invention may include other agents conventional in the art having regard to the type of formulation in question, for example, those suitable for oral administration may include flavoring agents.

The present invention includes compositions of about 100% to about 90% pure isomers. In another aspect, the invention pertains to compositions of about 90% to about 80% pure isomer. In yet another aspect, the invention pertains to compositions of about 80% to about 70% pure isomer. In still another aspect, the invention pertains to a composition of about 70% to about 60% pure isomer. In yet a further aspect, the invention pertains to a composition of about 60% to about 50% pure isomer. However, a steriochemical isomer labeled as alpha or beta may be a mixture of both in any ratio, where it is chemically possible by one skilled in the art. Additionally, included by this invention are both classical and non-classical bio-isosteric atom and substituent replacements and are well known by one skilled in the art. Such bio-isosteric replacements include, for example, substitution of =S or = NH for =O.

Known compounds that are used in accordance with the invention and precursors to novel compounds according to the invention can be purchased from commercial sources, for example, Sigma-Aldrich. Other compounds according to the invention can be synthesized according to known methods well known to those skilled in the art.

The synthetic route for benzo[c]chromen-6-one derivatives SG00292 and SG00392 are summarized in Scheme 1, *infra.* This synthetic route presents one potential way to prepare this series of derivatives, and other synthetic routes (including modifying the order of synthetic steps or reagents) are possible to someone skilled in the art. In specific cases, the nature of protecting groups or the order of reactions may have to be altered in order to reach the desired products. These changes to the general synthetic schemes are well understood to one skilled in the art.

### EXAMPLES

Benzo[c]chromen-6-one derivatives according to the present invention may be prepared using the following reaction schemes, Scheme 1 and synthetic methods Scheme 2.

The present invention also includes benzo[c]chromen-6-one derivatives prepared from the starting point of Scheme 1. The synthesis of these analogs are described in the synthetic methods shown in Scheme 3 and represents examples from the benzo[c]chromen-6-one derivatives as depicted in Table I.

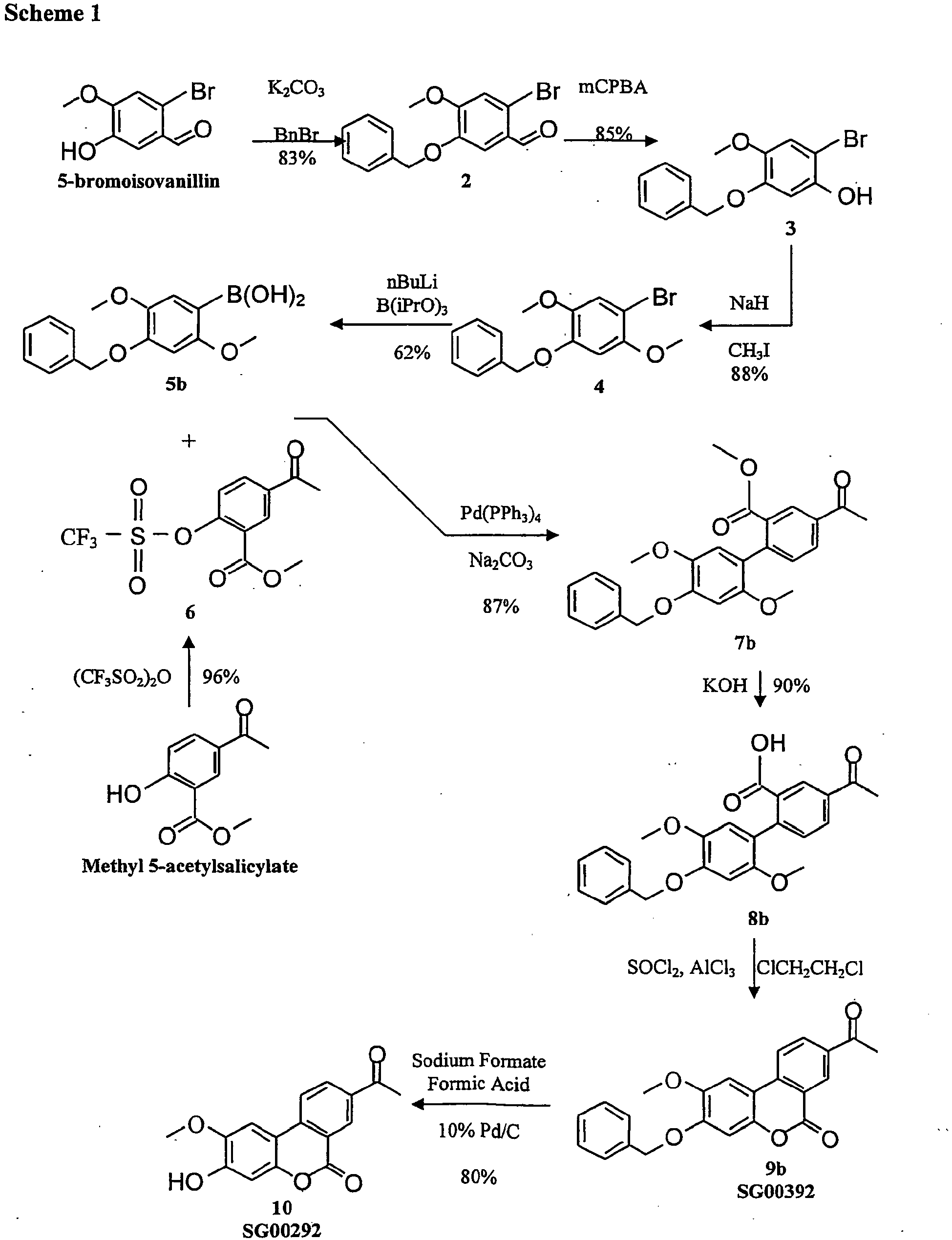

### Scheme 2

### Synthesis 5-Benzyloxy-2-bromo-4-methoxybenzaldehyde (2)

2-Bromo-5-hydroxy-4-methoxybenzaldehyde (25 g, 0.108 mol) and K₂CO3 (30 g, 0.216 mol) were added to acetonitrile (250 mL) and flushed with Ar. Benzyl bromide (20 g, 0.12 mol) was added and the mixture was heated under Ar for 20 h at 50°C. After cooling, the mixture was poured into water (200 ml) and extracted with CH₂Cl₂ (300 mL). The CH₂Cl₂ was washed with water (3 x 100 mL), dried and concentrated. Recrystallization with isopropanol: water (3:1) gave 28.8 g (83%) of 2 as a light brown solid. ¹H-NMR (400 MHz, CDC13) dH 3.96 (3H, s, OCH₃), 5.16 (2H, s, CH2Ph), 7.07-7.48 (7H, m, ArH + CH₂Ph), 10.16 (1H, s, CHO).

### 5-Benzyloxy-2-bromo-4-methoxyphenol (3)

5-Benzyloxy-2-bromo-4-methoxy-benzaldehyde 2 (5 g, 16.0 mmol) was added to CH2Cl2 (40 mL), flushed with Ar and cooled in an ice bath. A solution of mCPBA (5.2 g) in CH₂Cl₂ (50 mL) was added dropwise. Once the addition was complete the reaction mixture was refluxed under Ar for 14 h. After cooling the mixture was washed with sat. NaHCO₃ (3 x 50 mL), brine, dried and concentrated. The residue was recrystallized from ethyl acetate/hexanes to 4.1 g (85%) of 3 as large tan needles. ¹H-NMR (400 MHz, CDCl3) dH 3.88 (3H, s, OCH₃), 5.10 (2H, s, CH₂Ph), 6.74 (1H, s, ArH), 7.08 (1H, s, ArH), 7.34 -7.40 (5H, m, CH₂Ph), 8.25 (1H, s, OH).

### 1-Benzyloxy-4-bromo-2,5-dimethoxybenzene (4)

5-Benzyloxy-2-bromo-4-methoxy-phenol 3 (2.76 g, 89.0 mmol) and NaH (0.89 g, 13.0 mmol, 60% dispersion in oil) were added to a flask and flushed with Ar. Dry THF (50 mL) was added and the suspension was stirred in an ice bath for 20 min. CH₃I (1.7 mL, 27.0 mmol, filtered through basic alumina) was added and the mixture stirred at room temperature under Ar for 18 h. After cooling the reaction mixture in an ice bath, water was added slowly. The mixture was extracted with ethyl acetate, dried and concentrated to give yellow oil that solidified under vacuum. The oil was purified by silica gel chromatography using silica gel with (10% ethyl acetate/hexanes) to give 2.5 g (88%) of 4 as a white solid. ¹H-NMR (400 MHz, CDC13) dH 3.75 (3H, s, OCH₃), 3.84 (3H, s, OCH₃), 5.15 (2H, s, CH₂Ph), 6.57 (1H, s, ArH),7.07(1H,s,ArH), 7.32 -7.42(5H,m,CH₂Ph).

### 4-Benzyloxy-2,5-dimethoxyphenylboronic acid (5b)

1-Benzyloxy-4-bromo-2,5-dimethoxybenzene 4 (7.48g,23.0mmol) was placed in a dry flask and flushed with Ar. Dry THF (75 mL) was added and the solution was cooled to -78°C in a dry ice/acetone bath. nBuLi (11 mL, 2.5M in hexanes) was added and the mixture was stirred for 20 min at -78°C. Triisopropyl borate (10.7 mL, 0.463 mol) was added and the reaction stirred for 2h at -78°C then allowed to come to room temperature at which time a white precipitate began to form. After stirring for an additional 20 h the reaction was quenched with saturated NH₄Cl (25 mL). After separating the organic layer the aqueous layer was extracted with ethyl acetate (2 x 50 mL). The organic layers were combined, dried and concentrated. The residue was triturated with hexanes and filtered to give 4.1 g (62%) of 5b as a light off-white creamy solid. ¹H-NMR (400 MHz, DMSO-d6) dH 3.70 (3H, s, OCH₃), 3.79 (3H, s, OCH₃), 5.16 (2H, s, CH₂Ph), 6.77 (1H, s, ArH), 7.18 (1H, s, ArH), 7.33 -7.52 (5H, m, CH₂Ph)

### 5-Acetyl-2-trifluoromethanesulfonyloxybenzoic acid methyl ester (6)

Methyl 5-acetylsalicylate (25.0 g, 0.129 mol) was dissolved in CH₂Cl₂ (250 mL) and pyridine (60 mL) under Ar at 0°C. Trifluoromethanesulfonic anhydride (37.9 g, 0.133 mol) was then added over 20 min. The reaction mixture was stirred for an additional 30 min and then quenched with water (500 mL). The organic layer was separated and washed three times with 5% HCl (80 mL). After removing the solvent the solid obtained was dried under vacuum to yield 40.3 g (96%) of 6. ¹H-NMR (400 MHz, CDCl₃) dH 2.56 (3H, s, COCH₃), 3.89 (3H, s, OCH₃), 7.32 (1H, d, ArH), 8.12 (1H, d, ArH), 8.52 (1H, s, ArH),

### 4-Acetyl-4'-benzyloxy-2'-methoxybiphenyl-2-carboxylic acid methyl ester (7b)

4-Benzyloxy-2,5-dimethoxyphenylboronic acid 5b (4.15 g, 14.4 mmol), 5-Acetyl-2-trifluoromethanesulfonyloxy-benzoic acid methyl ester 6 (4.69g,14.4mmol) and K₂CO₃ (3.98 g, 28.8 mmol) were added and the flask was flushed with Ar. Absolute ethanol (83 mL) and DME (94 mL) were added followed by Pd (PPh₃)₄ (0.87 g, 0.785 mmol) and the reaction mixture refluxed for 4 h. After cooling, water (100 mL), ethyl acetate (100 mL) and brine (50 mL) were added. The organic layer was washed with brine (2 x 50 mL) and the combined aqueous fraction was back extracted with ethyl acetate. The combined organic fraction was dried, concentrated and recrystallized from ethyl acetate/hexanes to give 6.5 g (99%) of 7b as a yellow solid. ¹H-NMR (400 MHz, CDC13) dH 2.65 (3H, s, COCH₃), 3.58 (3H, s, OCH₃), 3.68 (3H, s, OCH₃), 3.88 (3H, s, OCH₃), 5.21 (21H, s, CH₂Ph), 6.55 (1H, s, ArH), 6.86 (1H, s, ArH), 7.30-7.48 (6H, m, ArH + CH₂Ph), 8.10 (1H, d, ArH),8.37(1H,s,ArH),

### 4-Acetyl-4'-benzyloxy-2'-methoxybiphenyl-2-carboxylic acid (8b)

To 4-Acetyl-4'-benzyloxy-2'-methoxybiphenyl-2-carboxylic acid methyl ester 7b (4.06 g, 9.7 mmol) and NaOH (0.773 g, 19.4 mmol) was added methanol (60 mL) and water (60 mL). The reaction was refluxed under Ar for 7 h then cooled to room temperature. After placing in an ice bath, 1 M HCl was added to give a yellow precipitate that was filtered, washed with water and recrystallized from THF/hexanes to give 2.7 g (69%) of 8b as yellow crystals. ¹H-NMR (400 MHz, CDCl₃) dH 2.68 (3H, s, COCH₃), 3.62 (3H, s, OCH₃), 5.16 (2H, s, CH₂Ph), 6.77 (1H, s, ArH), 7.18 (1H, s, ArH), 7.33 -7.52 (5H, m, CH₂Ph), 3.90 (3H, s, OCH₃), 5.22 (2H, S, CH₂Ph), 6.58 (1H, s, ArH), 6.90 (1H, s, ArH), 7.34 -7.50 (6H, m, ArH + Ch₂Ph), 8.17 (1H, d, ArH), 8.50 (1H, s, ArH),

### 8-Acetyl-3-benzyloxy-2-methoxybenzo[c]chromen-6-one (9b)

4-Acetyl-4'-benzyloxy-2'-methoxybiphenyl-2-carboxylic acid 8b (1.0 g, 2.5 mmol) was suspended in 1,2-dichloroethane (30 mL). SOCl₂ (200 mL, 2.7 mmol) was added and the reaction mixture refluxed for 2 h under Ar. After cooling to room temperature (some precipitate formed) AlC₁₃ (0.262 g, 0.002 mol) was added turning the mixture red. The reaction was stirred at room temperature for 17 h then quenched with water (30 mL) and diluted with CH₂Cl₂ (100 mL). After washing the organic layer with brine (2 x 50 mL) it was dried and concentrated. The residue was dissolved in hot CHCl₂ and then cooled. Hexanes were added to help precipitate the product. A second recrystallization gave 0.3 g (32%) of 9b as a yellow solid. ¹H-NMR (400 MHz, CDCl₃) dH 2.73 (3H, s, COCH₃), 4.05 (3H, s, OCH₃), 5.28 (2H, s, CH₂Ph), 6.94 (1H, s, ArH), 7.35-7.50 (6H, s, ArH+ CH₂Ph), 8.07 (1H, d, ArH), 8.42 (1H, d, ArH), 8.92 (1H, s, ArH)

### 8-Acetyl-3-hydroxy-2-methoxybenzo[c]chromen-6-one (10)

Sodium formate (2.18 g, 32 mmol) and formic acid (4.2 mL, 106.8 mmole) were added to a suspension of **9b** (10.0 g, 26.71 mmol) in a 1:1 mixture of dry THF and absolute ethanol (1.5 L) in a 3 liter 3-necked flask equipped with an overhead stirrer and a heating mantle. To this mixture was added 100 mg of 10% palladium on carbon and the reaction refluxed under argon for 7 hours. At this time, all of the starting 9b had gone into solution. The solution was filtered hot to remove the catalyst and the solvent removed by rotary evaporation. (If the solution is allowed to cool down, the product will precipitate and can be separated from the catalyst by extracting the solid with 6 liters of refluxing methanol). The resulting solid (7.8 g) was purified by silica gel chromatography as described below.

In a typical run, 3.12 g of crude **10** was mixed with 20 g of silica gel, suspended in 200 mL of methanol and the solvent removed by rotary evaporation. This material was placed on top of a silica gel column (6 cm x 36 cm , 400 g of silica gel), and eluted with a stepwise gradient of 1% acetone / dichloromethane, 10% acetone / dichloromethane and 100% acetone. All pure fractions were combined and evaporated to give 2.4 g (80% yield) of the desired intermediate **10**. ¹H (300 MHz) (DMSO-*d₆*) d 2.07 (3H, s), 2.66 (3H, s), 3.92 (3H, s), 6.81 (1H, s), 7.78 (1H, s), 8.33 (1H, d, J = 8.7 Hz), 8.46 (1H, d, J = 8.7 Hz) and 8.66 (1H, d, J = 1.8 Hz).

### Scheme 3

**SG00393.** SG00392 (1.0 g, 2.67 mmol) and NaBH₄ (0.1 g, 2.67 mmol) were added to a 2:1 mixture of THF (20 mL) and absolute ethanol (10 mL) and left to stir for 1.5 h. The reaction mixture was cooled in an ice bath and 0.5 N HCl added until the color changed from yellow to clear. Water (20 mL) was added and the mixture extracted with CH₂Cl₂, dried, concentrated and the residue purified by silica gel flash column chromatography using CH₂Cl₂:acetone (8/1) to give 0.71 g of SG00393.

**SG00394.** SG0093 (0.1 g, 0.27 mmol) was added to anhydrous CH₂Cl₂ (6 mL) and cooled to -78°C giving a heterogeneous mixture. DIBAL (1M in hexanes, 0.66 mL, 0.66 mmol) was added dropwise over 2 h. An additional amount of DIBAL was added (0.2 mL) and after a total time of 2.5 h the reaction was quenched by the addition of methanol (0.8 mL). The reaction mixture was allowed to come to room temperature, CH₂Cl₂ (100 mL), ice and a small amount of acetone were added and the mixture stirred for 15 min. The CH₂Cl₂ layer was washed with st NaHCO₃, brine, dried and concentrated. The residue was redissolved in acetone (40 mL) and pre-adsorbed onto silica gel (1 g). After evaporation of the acetone the residue was purified by silica gel flash column chromatography using CH₂Cl₂:acetone (6/1) to give 69 mg of SG0094.

**SG00395.** Crude SG00394 (1.18 g, 3.12 mmol), triethylamine (1.73 mL, 12.5 mmol), acetic anhydride (1.18 mL, 12.5 mmol) and anhydrous CH₂Cl₂ (50 mL) were stirred at room temperature under N₂. Once crystal of DMAP was added, the reaction mixture stirred for 15 min, then extracted with CH₂Cl₂. The CH₂Cl₂ layer was washed with sat NaHCO₃, brine, dried, concentrated and purified by silica gel flash column chromatography using CH₂Cl₂:acetone (10/1) to give 0.89 g of SG00395.

**SG00396.** SG00395 (0.83 g, 0.197 mmol) was added to anhydrous CH₂Cl₂ (25 mL) and cooled in a methanol/dry ice bath under N2. Et₃SiH (0.631 mL, 3.95 mmol) was added followed by BF₃ Et₂O (0.375 mL, 2.96 mmol) dropwise and stirred vigorously for 0.5 h. The reaction mixture was removed from the cooling bath and after 45 minutes quenched with sat NaHCO₃ (3 mL). The reaction mixture was extracted with CH₂Cl₂, washed with sat. NaHCO₃, brine, dried, concentrated and purified by silica gel flash column chromatography using ethyl acetate:hexanes (1/2) to give 0.71 g of SG00396.

**SG00397.** SG00396 (0.135 g, 0.334 mmol), formic acid (0.525 mL, 1.34 mmol), sodium formate (27 mg, 0.4 mmol), 10% Pd/C (0.3 mol %), anhydrous THF (4 mL) and absolute ethanol (4 mL) were heated to reflux under N₂ for 1.5 h. The reaction was cooled and approximately half of the reaction mixture evaporated. The silica gel residue was purified by silica gel flash column chromatography using ethyl acetate:hexanes (1/2) to give 50 mg of SG00397.

**SG00398.** To the remaining half of the reaction mixture in the preparation of SG00397 was added additional 10% Pd/C and the reaction refluxed for 0.5 h. The Pd/C was filtered off, washed with methanol and silica gel added to the filtrate. After concentrating, the silica gel residue was purified by silica gel flash column chromatography using ethyl acetate:hexanes (1/2) to give 32 mg of SG00398.

**SG00399.** SG00395 (0.44 g, 1.09 mmol) and Amberlyst-15 resin (12-15 beads) were stirred in methanol (10 mL) under N₂ for 2 h. The Amberlyst was filtered, washed with methanol and the filtrate concentrated. The residue was purified by silica gel flash column chromatography using ethyl acetate:hexanes (1/2) to give 0.4g of SG00399.

**SG00400.** SG00397 (95 mg, 0304 mmol) was added to methanol (2 mL). To this mixture K₂CO₃ (0.126 g, 0.912 mmol) and water (0.1 mL) were added and the reaction stirred under N₂ for 3 h. The reaction was stopped by the addition of 1% HCl (0.1 mL) and methanol (10 mL). Silica gel was added, the solvent evaporated and the residue was purified by silica gel flash column chromatography using ethyl acetate:hexanes (1/1) to give 72 mg of SG00400.

**SG00477.** SG00292 (0.18 g, 0.63 mmol) was added to anhydrous CH₂Cl₂ (7 mL) with stirring under N₂. Et₃N (0.35 mL, 2.53 mmol), acetic anhydride (0.24 mL, 2.53 mmol) and one crystal of DMAP were added. After stirring for 15 min. CH₂Cl₂ was added and the mixture washed with sat NaHCO₃, brine, dried, concentrated and pre-adsorbed onto silica gel. The silica gel flash column chromatography using ethyl acetate:hexanes (2/1) to give 80 mg of SG00477.

**SG00490.** SG00396 (122 mg, 0.3 mmol), K₂CO₃ (125 mg, 0.9 mmol) and water (0.13 mL) were added to methanol (3.3 mL) and stirred under N₂ for 1.5 h then quenched with 1% H₂SO₄. The reaction was extracted with CH₂Cl₂ and divided into two equal portions. One portion was concentrated and purified by silica gel flash column chromatography using ethyl acetate:hexanes (1/1) to give 48 mg of SG00490. The remaining portion was converted to SG00491.

**SG00491.** The remaining portion of crude SG00490 was oxidized using the Dess-Martin reagent (37.3 mg, 0.9 mmol) over 1 h. The reaction was extracted with CH₂Cl₂, washed with sat NaHCO₃, brine, dried, concentrated and purified by silica gel flash column chromatography using ethyl acetate:hexanes (1/1) to give 40 mg of SG00491.

**SG00492.** Prepared following the method for SG00392 starting with SG00491. Yield 44 mg.

**SG493.** SG492 (116 mg, 0.41 mmol), K₂CO₃ (112 mg, 0.82 mmol) and CH₃I (1 mL) were added to acetone (10 mL) and refluxed for 2 days. Silica gel was added to the reaction mixture, concentrated and purified by silica gel column chromatography using silica gel flash column chromatography using CH₂Cl₂:acetone (9/1) to give 100 mg of SG00493.

**SG00494.** Prepared following method for SG00493 using 1-(2-chloroethyl)piperidine hydrochloride. Yield 52 mg.

**SG00495.** Prepared following method for SG00493 using ethyl bromide. Yield 20 mg.

**SG00496.** SG00393 (116 mg, 0.308 mmol) was added to anhydrous THF (10 mL) in an ice bath. NaH (60% dispersion in oil, 22 mg, 0.92 mmol) was added and the mixture stirred for 20 min. CH₃I was added dropwise and the reaction stirred for 0.5 h. The ice bath was removed and the reaction was stirred overnight. Additional CH₃I was added and the reaction mixture refluxed for 5 h. The reaction was quenched with water and distilled to remove the excess CH₃I. CH₂Cl₂ and water were added, and after separating, the CH₂Cl₂ layer was dried, concentrated and purified by silica gel flash column chromatography using ethyl acetate:hexanes (1/1) to give SG00496.

**SG00510.** Prepared following the method for SG00393 using SG00493. Yield 48 mg.

**SG00511**. Prepared following the method for SG00493 using 2-(bromomethyl) hydrobromide. Yield 170 mg.

**SG00512.** Prepared following the method for SG00493 using ethyl bromide. Yield 63 mg.

**SG00513.** Prepared following the method for SG00493 using isopropyl bromide. Yield 220 mg.

**SG00514.** Prepared following the method of SG00493 using 7-hydroxycourmarin and benzyl bromide. Yield 1.3 g.

**SG00519.** Prepared following the method for S000493 using scopoletin and benzyl bromide. Yield 17 mg.

**SG00520.** Prepared following the method for SG00393 using SG00494. Yield 75 mg.

**SG00521.** Prepared following the method for SG00393 using SG00512. Yield 118 mg.

**SG00526.** SG00511 (50 mg, 0.133 mmol) and NaBH₄ (5.0 mg, 0.133 mmol) were added to a 1:1 mixture of ethanol and THF (10 mL total) and left to stir for 48 h, then refluxed for 2 h. After cooling the reaction mixture was acidified to pH 2 with 1 N HCl then taken to pH 8 with sat NaHCO₃ and extracted with ethyl acetate (3 x 20 mL). The combined organic layers were washed with water, brine, dried and concentrated. The residue was purified by silica gel chromatography using a gradient of hexanes:CHCl₃ (1/1) following by CHCl₃ following by 3% CH₃OH/CHCl₃ to give 40 mg of SG00526.

**SG00527.** SG292 (100 mg, 0.35 mmol) was added to a mixture ofNaH (15.4 mg, 0.4 mmol) in DMF (10 mL) and the reaction mixture refluxed for 2 h. After cooling down to room temperature 4-methoxybenzyl bromide (0.57 mL, 0.42 mmol) dissolved in DMF was added and the reaction mixture heated to 70°C for 9 h. Water (10 mL) was added and the reaction mixture extracted with CHCl₃ (3 x 20 mL), the combined organic layers were washed with water, brine, dried and concentrated. The residue was purified by hexanes:CHCl₃ (1/2) followed by CHCl₃ to give 85 mg of SG00527.

**SG00528.** Prepared following method for SG00526 using SG00530. Yield 20 mg.

**SG00529.** Prepared following method for SG00526 using SG00527. Yield 40 mg.

**SG00530.** Prepared following method for SG00527 using 3-methoxybenzyl bromide. Yield 110 mg.

**SG00531.** Prepared following method for SG00393 using SG00495. Yield 36 mg.

**SG00532.** Prepared following method for SG00393 using SG00513. Yield 71 mg.

**SG00533.** Prepared following method for SG00393 using SG00273. Yield 8 mg.

**SG00541.** Prepared following method for SG00527 using 2-methoxybenzyl chloride. Yield 80 mg.

**SG00542.** Prepared following method for SG00527 using 2-(chloromethyl)phenyl acetate. Yield 80 mg.

**SG00543.** To SG00392 (0.19 g, 0.51 mmol) in anhydrous CH₂Cl₂ (8 mL) was added CH₃MgI (0.2 mL, 1.6 mM) dropwise with stirring at room temperature under N₂. After 25 min additional CH₃MgI (0.2 mL, 1.6 mM) was added. After 1 h still additional CH₃MgI (0.2 mL, 1.6 mM) was added. The CH₂Cl₂ was separated, washed with slightly acidic water, silica gel added and the CH₂Cl₂ evaporated to pre-adsorb the crude reaction. The dimethyl alcohol was purified by silica gel flash column chromatography using ethyl acetate:hexanes (2/1) and used in the next step. The dimethyl alcohol (58 mg, 0.15 mmol) was debenzylated following the method for SG00292 to give SG00543. Yield 32 mg.

**SG00544.** Prepared following method for SG00526 using 2-chloromethylphenyl acetate. Yield 15 mg.

**SG00545.** Prepared following method for SG00526 starting with SG00541. Yield 40 mg.

**SG00546.** Prepared following method for SG00527 using 3,5-dimethoxybenzyl chloride. Yield 80 mg.

**SG00547.** Prepared following method for SG00526 starting with SG00546. Yield 30 mg.

**SG00548.** The dimethyl alcohol (56 mg, 0.14 mmol) produced in the preparation of SG00543 was added to anhydrous CH₂Cl₂ (3 mL) containing a catalytic amount of Amberlyst-15 and MgSO₄ and stirred for 6 h and then placed in the freezer overnight. After filtering, the crude dehydration product was purified by silica gel flash column chromatography using ethyl acetate:hexanes (1/2) and used in the next step. The purified dehydration product was dissolved in absolute ethanol (3 mL) and a suspension of 10% Pd-C (30 mg) in absolute ethanol (1.5 mL) was added and a balloon filled with H₂ attached. After stirring for 7 h the catalyst was filtered off, the crude reaction pre-adsorbed onto silica gel and purified by silica gel flash column chromatography using ethyl acetate:hexanes (1/2) to give SG00548.

**SG00549.** To a suspension ofNaH (0.02 g, 0.55 mmol) in anhydrous DMF (5 mL) was added SG00391 (0.1 g, 0.37 mmol). The resulting yellow opaque mixture was refluxed for 1 h. Benzyl bromide (0.05 mL, 0.41 mmol) was added and the mixture became an orange/yellow clear solution. The reaction mixture was cooled added to water (15 mL) and extracted with ethyl acetate (3 x 12 mL). The organic layer was washed with brine, dried, concentrated and purified by flash silica gel chromatography using 15% ethyl acetate in hexanes to give SG00549 in a quantitative yield.

**SG00550.** Prepared following method for SG00549 using 4-methoxybenzyl bromide. Yield 100 mg.

**SG00551.** Prepared following method for SG00549 using 2-methoxybenzyl bromide. Yield 62 mg.

**SG00552.** Prepared following method for SG00549 using 3-methoxybenzyl bromide. Yield 70 mg.

**SG00553.** Prepared following method for SG00526 starting with SG00555. Yield 20 mg.

**SG00554.** Prepared following method for SG00526 starting with SG00556. Yield 24 mg.

**SG00555.** Prepared following method for SG00527 using 3-chloromethylpyridine hydrochloride. Yield 53 mg.

**SG00556.** Prepared following method for SG00527 using 4-chloromethylpyridine hydrochloride. Yield 45 mg.

**SG00557.** Prepared following method for SG00527 using 4-(chloromethyl)phenyl acetate. Yield 5 mg.

**SG00558.** Prepared following method for SG00527 using 4-(chloromethyl)phenyl. Yield 45 mg.

**SG00559.** Prepared following method for SG00527 using 4-methylbenzyl bromide. Yield 58 mg.

**SG00560.** Prepared following method for SG00549 using 4-bromobenzyl bromide. Yield 60 mg.

**SG00561.** Prepared following method for SG00549 using 3-bromobenzyl bromide. Yield 100 mg.

**SG00562.** Prepared following method for SGO0549 using 3-chlorobenzyl bromide. Yield 80 mg.

**SG00568.** Prepared following method for SG00527 using 2-bromoethyl benzene. Yield 18 mg.

**SG00569.** Prepared following method for SG00543 using PhMgBr. Yield 19 mg.

**SG00570.** Prepared following the preparation of the dehydration product in the synthesis of SG00548 using the diol side product generated in the preparation of SG00549. Yield 21 mg.

**SG00571.** Prepared following method for SG00549 using 4-chlorobenzyl bromide. Yield 90 mg.

**SG00572.** Prepared following method for SG00549 using 4-flurobenzyl bromide. Yield 110 mg.

**SG00573.** Prepared following method for SG00549 using methyl 4-(bromomethyl)benzoate. Yield 40 mg.

**SG00574.** Prepared following method for SG00549 using 4-bromomethyl benzophenone. Yield 30 mg.

**SG00575.** Prepared following method for SG00526 starting with SG00559. Yield 25 mg.

**SG00576.** Prepared following method for SG00527 using 3-methylbenzyl bromide. Yield 30 mg.

**SG00577.** Prepared following method for SG00527 using 3,4,5-trimethoxybenzyl bromide. Yield 45 mg.

**SG00592.** Prepared following method for SG00527 using 4-methoxybenzyl chloride and 3-)4-bromophenyl)-7-hydroxycoumarin. Yield 62 mg.

**SG00593.** Prepared following method for SG00592 using 3,5-dimethoxybenzyl bromide. Yield 74 mg.

**SG00594.** Prepared following method for SG00527 using 4-trifluromethylbenzyl chloride. Yield 22 mg.

**SG00595.** Prepared following method for SG00527 using 4-fluorobenzyl chloride. Yield 43 mg.

**SG00596.** Prepared following method for SG00549 using 3,5-dimethoxybenzyl bromide. Yield 30 mg.

**SG00597.** Prepared following method for SG00527 using ethyl bromoethyl acetate. Yield 15 mg.

**SG00598.** Prepared following method for SG00527 using SG00293 (the ketone of SG00292 reduced to the alcohol). Yield 16 mg.

**SG00599.** From the reaction to prepare SG00569, SG00599 was also isolated. Yield 3.3 mg.

**SG00609.** Prepared following method for SG00526 starting with SG00577. Yield 32 mg.

**SG00612.** SG00292 (0.1g, 0.35 mmol) was added to anhydrous CH₂Cl₂ (10 mL) with stirring. Pyridine (0.05 mL) and benzoyl chloride (0.1 mL) were added and the reaction stirred for 1 h. The reaction was poured into 5% HCl, extracted with CH₂Cl₂, washed with sat NaHCO₃, dried, concentrated and purified by flash silica gel chromatography using ethyl acetate:hexanes (1/1) to give 25 mg of SG00612.

**SG00613.** Prepared following method for SG00612 using 4-methoxybenzyl chloride. Yield 2.3 mg.

**SG00614.** SG00547 (50 mg, 0.114 mmol) was dissolved in a 1:1 mixture of anhydrous diethyl ether and CH₂Cl₂ (6 mL). PBr₃ (124 mg, 0.46 mmol) was added and the reaction. stirred over the weekend at room temperature. Sat NaHCO₃ was added and the reaction extracted with CH₂Cl₂, washed with brine, dried, concentrated and purified by flash silica gel chromatography using hexanes then CHCl₃ then 1% methanol in CHCl₃ to give 20 mg of SG00614.

**SG00615.** Prepared following the method for SG00543 starting with SG00546 and EtMgBr. Yield 55 mg.

**SG00616.** Prepared following the method for SG00543 starting with SG00546 and CH₃MgI. Yield 74 mg.

**SG00617.** Prepared following method for SG00527 using SG00293 (the ketone of SG00292 reduced to the alcohol) and 4-bromomethyl benzophenone. Yield 13 mg.

**SG00618.** 4-benzyloxybenzoic acid (1 g, 4.4 mmol) was added to anhydrous CH₂Cl₂ (11 mL). A catalytic amount of DMF (5 drops) was added along with oxalyl chloride in CH₂Cl₂ (2M, 5.75 mL) and the reaction stirred for 2 h. The solvents were evaporated and the crude 4-benzyloxybenzoyl chloride was used directly. SG00618 was prepared following the method for SG00612 using 4-benzylbenzoyl chloride. Yield 49 mg.

**SG00619.** Prepared following the method of SG00527 but using SG00293 (the methyl ketone of SG00292 reduced to the alcohol) and 4-formylbenzyl bromide (prepared by DIBAL reduction of 4-cyanobenzyl bromide. Yield 45 mg.

**SG00620.** Prepared following the method for SG00527 using 4-nitrobenzyl bromide. Yield 20 mg.

### Experimental Data

The following examples refer to representative illustrations from the compounds depicted in Table I. The aforementioned compounds are found to have anti-proliferative, anti-angiogenic properties and/or other meaningful activities to be described below.

### Example 1: Anti-angiogenic activity measured in vitro as inhibition of proliferation of endothelial cells and lack of binding to estrogen receptor alpha and beta

HUVEC Proliferation. Inhibition of the proliferation of human umbilical vein endothelial cells, HUVECs, is shown as one measure of anti-angiogenic activity. HWECS and the required media complements were purchased from Cascade Biologies (Portland, OR) and the growth and maintenance of the cultures was as described by the manufacturer. The proliferation assay was carried out by seeding the HUVECs in 96-well plates at a density of 1,000 cells/well in complete medium. Following a 24 h plating period, the cells were starved for 24 h in 0.5% serum before being treated with SG ("Signal Gene" now "Palomid") angiogenic inhibitors in the presence of 10 ng/ml b-FGF or dosing ranging presence of either b-FGF or VEGF in complete medium. After 48 h, cell number was determined using a calorimetric method as described by the supplier (Promega Corp., Madison, WI). The results were expressed as the percentage of the maximal b-FGF or VEGF response in the absence of angiogenic inhibitors. Non-proliferating endothelial cells were assayed by growing HUVECs to quiescence in 96-well plates and treating with angiogenic inhibitors for 48 h. Initially, 5,000 cells/well were seeded and confluence was achieved the next day. The plates were incubated another 24 h to ensure growth arrest before treatment with angiogenic inhibitors. Cell number was determined as outlined above.

ER Binding Assay. Derivatives which bind and transduce a signal through estrogen receptors would not be considered a positive activity as such an activity could enhance cancer growth as well as induce angiogenesis. Derivatives which either have little or no binding to estrogen receptors ("ER") would be one desired activity. Alternatively, derivatives which bound to estrogen receptors but did not transduce a signal could also be considered a positive activity. Human cDNAs encoding ERa and ERb were used as templates to express receptor proteins *in vitro.* The proteins were produced with rabbit reticulocyte lysates as supplied by Promega (TNT kit) that couples transcription and translation in a single reaction. The amount of template used in each reaction was determined empirically and expression was monitored in parallel reactions where [³⁵S]methionine was incorporated into the receptor followed by gel electrophoresis and exposure to film. Binding reactions were carried out in 100 mL final volumes in TEG buffer (10 mM tris, pH 7.5, 1.5 mM EDTA, 10% glycerol). Five (5) mL of *in vitro* transcribed-translated receptor was used in each binding reaction in the presence of 0.5 nM

[³H]estradiol (E₂). All compounds were routinely tested from 10⁻¹¹ M to 10⁻⁶ M and were diluted in ethanol. The reactions were incubated at 4°C overnight and bound E₂ was quantified by adding 200 mL dextran-coated charcoal. After a 15 min rotation at 4 °C, the tubes were centrifuged for 10 min and 150 mL of the supernatant was added to 5 mL scintillation cocktail for determination of cpms by liquid scintillation counting. Controls for background were included in each experiment using 5 mL unprogrammed rabbit reticulocyte lysate. This value, typically 10 - 15% of the maximal counts was subtracted from all values. The maximum binding was determined by competing bound E₂ with only the ethanol vehicle. This value was set to 100% (maximal E₂ binding). Values for percent inhibition were calculated based on the maximal E₂ binding. The data were plotted and Ki values calculated using the Prism Software. Experiments were conducted at least three times in duplicate.

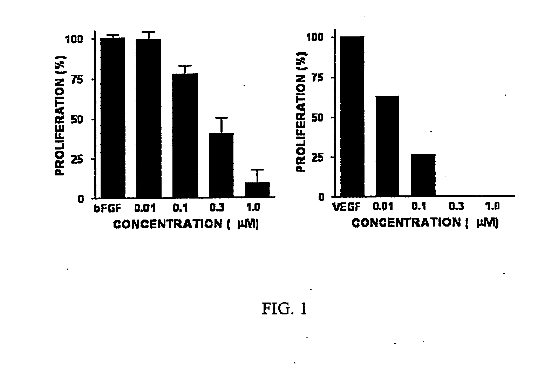

The results are shown in Table II and Figure 1. Activity of derivatives show anti-angiogenic activity through inhibition of the proliferation of angiogenic cytokine stimulated endothelial cells. The majority of the derivatives lack the ability to bind to estrogen receptors alpha and beta hence would not be expected to signal through these receptors, a possible stimulator of angiogenesis.

| **Table II** | | | | | |
|---|---|---|---|---|---|
| | **HUVECp** | **HUVECp** | **HUVECq** | **hERa % binding** | **hERb % binding** |
| **Palomid** | **% inhibition** | **% inhibition** | **% inhibition** | | |
| | **3 mM** | **0.3 mM** | **3 mM** | | |
| **529** | 113 | 65 | 31 | na | na |
| **547** | 106 | 42 | 25 | na | na |
| **575** | 104 | 41 | 33 | na | na |
| **545** | 100 | 32 | 22 | na | na |
| **528** | 80 | <10 | 25 | 41 | 31 |
| **550** | 77 | nd | 14 | na | na |
| **574** | 74 | 13 | 29 | na | na |
| **393** | 71 | nd | 21 | na | na |
| **551** | 62 | nd | na | na | na |
| **573** | 145 | nd | <10 | na | na |
| **546** | 100 | 18 | 23 | na | na |
| **559** | 96 | 72 | 35 | na | na |
| **568** | 78 | nd | 14 | na | 37 |
| **560** | 53 | nd | na | na | na |

| | | | | | |
|---|---|---|---|---|---|
| "na", no activity; HUVECp, HUVEC proliferating; HUVECq, HUVEC quiescent; hERa, human estrogen receptor alpha; hERb, human estrogen receptor beta | | | | | |

### Example 2: Apoptotic activity of derivatives

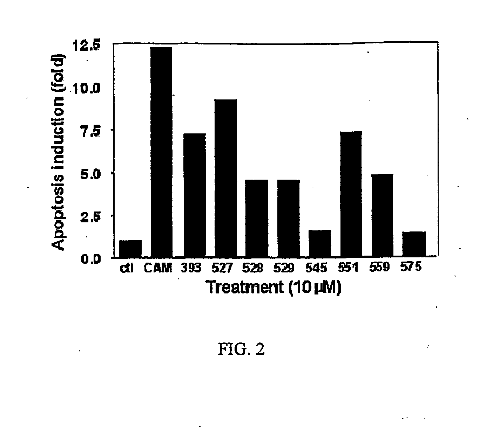

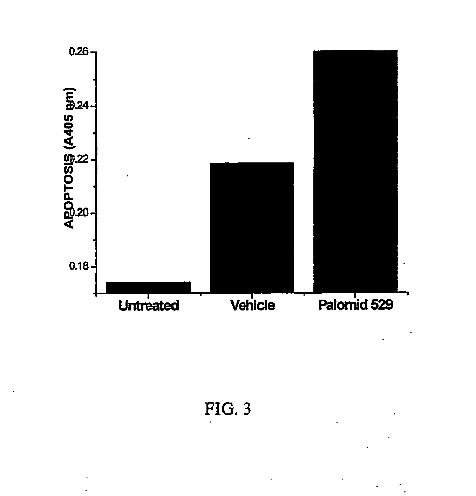

Apoptosis Assay. The apoptosis assay was conducted to determine if the derivatives inhibited cellular proliferation by inducing programmed cell death. Representative apoptotic activity is shown for endothelial cells with activity implied for other proliferating cells such as keratinocytes. Apoptosis of endothelial cells is yet another means to show anti-angiogenic activity. Cell death is monitored by quantifying the amount of cytoplasmic histone-associated DNA fragments that accumulate in the cell. Apoptosis assay kit was supplied by Roche (cat # 1 544 675) with ELISA detection and a monoclonal anti-histone antibody. Briefly, HUVECs or keratinocytes were trypsinized, diluted, and aliquoted into microfuge tubes at a concentration of 50,000 cells/tube. Treatment with a compound was for six hours at 37°C followed by cell lysis and analysis using the detection kit according to the manufacturer. Apoptosis was quantified calorimetrically at an absorbance of 405 nm. Controls consisted of a negative vehicle (ctl) control (1% ethanol) and a positive camptothecin (CAM) control at 4 mg/mL in ethanol. For keratinocyte assay, Palomid 529, a leading clinical candidate available from Paloma Pharmaceuticals, was added at 100 µM. Results are shown in Figure 2 and 3. (Palomid 529 is "SG00529", see Table I).

### Example 3: Anti-keratinocyte activity measured in vitro as inhibition of proliferation of keratinocytes and induction of apoptosis

Skin diseases at least in part are due to abnormal presence and proliferation of keratinocytes. Means to either inhibit said keratinocyte proliferation and/or the ability to induce apoptosis of keratinocytes in said diseases would be expected to aid in the amelioration of abnormal skin pathologies. The following represents illustrative data to support this supposition.

Keratinocyte proliferation. A benzo[c]chromen-6-one derivative, Palomid 529, was examined with the following protocol. Low passage human keratinocytes (NHEK, neonatal-pooled, p3-5, Cambrex, CC2507) were seeded in black 96-well plates at 1,000 cells per well in complete media (KBM, Cambrex) and incubated overnight. Cell culture media was then removed and replaced with fresh growth media plus either Palomid 529 or vehicle (1% DMSO). Palomid 529 was examined at nine concentrations (1:2 dilutions starting at 100 µM). Each experimental and control group was examined in replicates of six. Cultures were maintained for 3 days and cellular proliferation was then analyzed by determining metabolically active cells with a fluorescence-based assay (Alamar Blue, Invitrogen, diluted 1:20 in growth media, read following a five hour incubation, 530ex/580em).

Keratinocyte apoptosis. Palomid 529 was examined with the following protocol. Low passage human keratinocytes (NHEK, neonatal-pooled, p3-5, Cambrex, CC2507) were seeded in black 96-well plates at 3,000 cells per well in complete media (KBM, Cambrex) and incubated overnight (same plate as cell viability assay, adjacent wells). Cell culture media was then removed and replaced with fresh growth media plus either Palomid 529 or vehicle (1% DMSO). Palomid 529 was examined at seven concentrations (100, 30, 10, 1, 0.3, 0.1, & 0.03 µM). Each experimental and control group was examined in replicates of three. Following an overnight incubation, culture media was removed and the cells lysed with lysing reagent supplied with the Roche Cell Death ELISA plus kit. Lysed cells were then transferred to clean eppendorf tubes and centrifuged at 200 x g to remove nuclei and cell debris. Supernatants, containing the cytoplasmic fraction (including cytoplasmic nucleosomes), were then transferred to streptavidin coated plates and incubated with anti-Histone (biotin conjugated) and anti-DNA (POD conjugated) antibodies for two hours. Wells were then carefully washed. ABTS was then added to the wells and following the development of color (approximately 30 minutes), the reaction stopped by the addition of ABTS Stop solution. Apoptosis was then measured by reading the OD at 405nm with a reference background wavelength of 490nm.

## Claims

1. A benzo(c)chromen-6-one derivative selected from compound SG00529 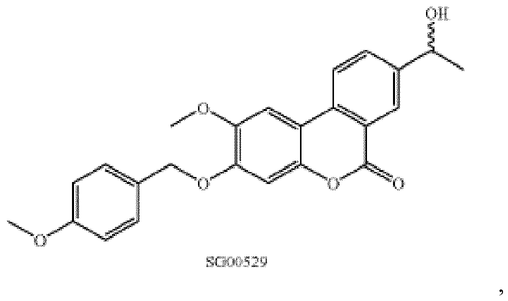 , and
compound SG00574 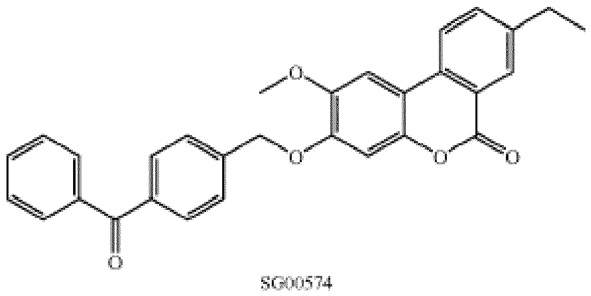 in free or salt form.

2. The benzo(c)chromen-6-one derivative of claim 1 in pharmaceutically acceptable salt form.

3. A pharmaceutical composition comprising a benzo(c)chromen-6-one derivative of claim 1.

4. The pharmaceutical composition of claim 3 further comprising a pharmaceutically acceptable carrier, diluent or excipient.

5. The pharmaceutical composition of any one of claims 3 - 4 further comprising one or more emollients.

6. The pharmaceutical composition of any one of claims 3 - 5 further comprising a thickener.

7. The pharmaceutical composition of any one of claims 3 - 6 further comprising an anti-inflammatory agent.

8. The pharmaceutical composition of any one of claims 3 - 7, wherein the composition is formulated in a biodegradable or non-biodegradable format for sustained release.

9. The pharmaceutical composition of any one of claims 3 - 8 formulated to be administered via oral, rectal, nasal, inhalation, topical, dermal, transdermal, buccal, sublingual, vaginal, parenteral subcutaneous, intramuscular, intravenous, intradermal, and inhalation administration.

10. The pharmaceutical composition of any one of claims 3 - 8 which is in the form of a capsule, cachet, or tablet.

11. The pharmaceutical composition of any one of claims 3 - 8 which is in the form of a solution, suspension, an oil-in-water liquid emulsion, or a water-in-oil emulsion.
